# EUROPEAN PATENT APPLICATION

(11) **EP 4 477 146 A1**
(43) Date of publication of application: **18.12.2024**
(21) Application number: 24181802.0
(22) Date of filing: 12.06.2024
(51) Int. Cl.: A61B 5/145, A61B 5/00, G16H 50/00

(54) **METHOD OF PROVIDING BEHAVIORAL INFORMATION FOR CONTROLLING GLUCOSE LEVEL, DEVICE FOR PERFORMING THE SAME, AND ANALYTE MONITORING SYSTEM**

(30) Priority: 14.06.2023 KR 20230076184
(71) Applicant: I-SENS, INC., Seoul 06646 (KR)
(72) Inventor: NAM, Hakhyun, 06646 Seoul (KR); CHO, Heegyung, 06646 Seoul (KR)
(74) Representative: Westphal, Mussgnug & Partner Patentanwälte mbB

(57) **Abstract**

A method of providing behavioral information for glucose level control according to an embodiment of the present disclosure includes: acquiring subject glucose level data corresponding to a hypoglycemic range through an analyte monitoring device; acquiring target glucose level data belonging to a normal glucose level range; acquiring a trained neural network model that calculates the behavioral information for changing a glucose level value corresponding to the hypoglycemic range to the normal glucose level range; acquiring the behavioral information including at least one of a type and amount of food to be consumed by a user from the subject glucose level data and the target glucose level data using the trained neural network model; and outputting the behavioral information through the user terminal device.

## Description

### BACKGROUND

### 1. Technical Field

The present disclosure relates to analyte monitoring, and more particularly, to technology for measuring a glucose level from an analyte using continuous glucose monitoring (CGM). Specifically, the present disclosure relates to a method of providing behavioral information for glucose level control, a device for performing the same, and an analyte monitoring system.

### 2. Related Art

A continuous glucose monitoring system (CGMS) is a system that acquires a glucose concentration of a user using a sensor in contact with a body fluid (e.g., interstitial fluid) of the user and provides the acquired glucose concentration to the user. The continuous glucose monitoring system includes a continuous glucose meter attached to the body of a user to sense signals from the body fluid of the user, and a user terminal device that provides the glucose concentration to the user.

The conventional continuous glucose monitoring system provides a low glucose level notification to a user through the user terminal device when the glucose concentration is outside the normal glucose range and detected in a low glucose range. However, the related art simply provides a user with only information that the low glucose level was measured, and is not able to track whether the glucose level of the user was approximately managed after a symptom of hypoglycemia was detected. Accordingly, the related art has a limitation in that the risk of shock due to hypoglycemia still remains.

Therefore, there is a need for technology development and research on an improved glucose level monitoring system to relieve hypoglycemia symptoms.

### SUMMARY

The present disclosure provides a method of providing information that is substantially helpful in relieving hypoglycemia symptoms, a device for performing the same, and an analyte monitoring system.

Problems to be solved by the present disclosure are not limited to the above-described objects, and objects that are not mentioned will be clearly understood by those skilled in the art to which the present disclosure pertains from the present specification and the accompanying drawings.

According to an embodiment of the present disclosure, a method of providing behavioral information for glucose level control by an analyte monitoring system includes: acquiring subject glucose level data corresponding to a hypoglycemic range through an analyte monitoring device; acquiring target glucose level data belonging to a normal glucose level range; acquiring a trained neural network model that calculates behavioral information for changing a glucose level value corresponding to the hypoglycemic range to the normal glucose level range; acquiring behavioral information including at least one of a type and amount of food to be consumed by a user from the subject glucose level data and the target glucose level data using the trained neural network model; and outputting the behavioral information through the user terminal device, wherein the trained neural network model may be configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and the trained neural network model may be trained by updating parameters of the neural network model to output the behavioral prediction value approximated to the behavioral data based on a difference between the behavioral prediction value and the behavioral data.

According to an embodiment of the present disclosure, an analyte monitoring device includes: a memory storing at least one instruction; and a processor, wherein the processor may execute the at least one instruction to acquire subject glucose level data corresponding to a hypoglycemic range, acquire target glucose level data belonging to a normal glucose level range, acquire a trained neural network model that calculates behavioral information for changing a glucose level value corresponding to the hypoglycemic range to the normal glucose level range, acquire behavioral information including at least one of a type and amount of food to be consumed by a user from the subject glucose level data and the target glucose level data using the trained neural network model, and transmit the behavioral information, the trained neural network model may be configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and the trained neural network model may be trained by updating parameters of the neural network model to output the behavioral prediction value approximated to the behavioral data based on a difference between the behavioral prediction value and the behavioral data.

Technical solutions of the present disclosure are not limited to the above-described solutions, and solutions that are not mentioned will be clearly understood by those skilled in the art to which the present disclosure pertains from the present specification and the accompanying drawings.

### BRIEF DESCRIPTION OF DRAWINGS

FIG. 1 is a schematic diagram illustrating an analyte monitoring system according to an embodiment of the present disclosure.
FIG. 2 is a block diagram illustrating a configuration of an analyte monitoring system according to an embodiment of the present disclosure.
FIG. 3 is a diagram for describing the operation of an analyte monitoring system and/or an analyte monitoring device according to an embodiment of the present disclosure.
FIG. 4 is a flowchart illustrating a method of providing behavioral information for glucose level control according to an embodiment of the present disclosure.
FIG. 5 is a flowchart illustrating a method of training a neural network model configured to calculate behavioral information for glucose level control according to an embodiment of the present disclosure.
FIG. 6 is a diagram illustrating an aspect of training a neural network model according to an embodiment of the present disclosure.
FIG. 7 is a diagram illustrating an aspect of calculating behavioral information using a trained neural network model according to an embodiment of the present disclosure.
FIG. 8 is a diagram illustrating an aspect of outputting a template corresponding to behavioral information according to an embodiment of the present disclosure.
FIG. 9 is a diagram illustrating an aspect of calculating behavioral information based on preference type information according to an embodiment of the present disclosure.
FIG. 10 is a diagram illustrating an aspect of providing a notification indicating that the glucose level has returned to a normal range or calculating additional behavioral information, depending on whether glucose level data after behavior has reached target glucose level data according to an embodiment of the present disclosure.
FIG. 11 is a diagram illustrating an aspect of training a neural network model whose training has been completed in a process of executing the neural network model according to an embodiment of the present disclosure.
FIGS. 12A and 12B are diagrams for describing aspects of determining whether behavior based on behavioral information has been performed according to an embodiment of the present disclosure.

### DESCRIPTION OF EXAMPLE EMBODIMENTS

Objects, features, and advantages of the present disclosure will become more obvious from the following detailed description provided in conjunction with the accompanying drawings. However, since the present disclosure may be variously modified and have several exemplary embodiments, specific exemplary embodiments of the present disclosure will be illustrated in the accompanying drawings and described in detail below.

In principle, the same reference numerals denote the same constituent elements throughout the specification. Further, elements having the same function within the scope of the same idea illustrated in the drawings of each embodiment will be described using the same reference numerals, and overlapping descriptions thereof will be omitted.

When it is determined that a detailed description for the known functions or configurations related to the present disclosure may obscure the gist of the present disclosure, detailed descriptions thereof will be omitted. In addition, numbers (for example, first, second, etc.) used in the process of describing the present specification are only identifiers for distinguishing one component from other components.

In addition, the suffixes "module" and "unit" for components used in the following embodiments are used only to facilitate the writing of the present disclosure. Therefore, these terms do not have distinct meanings or roles in themselves.

In the following embodiments, singular forms include plural forms unless the context clearly dictates otherwise.

In the following embodiments, the terms "include," "have," or the like means that a feature or element described in the specification is present, and it does not preclude the possibility that one or more other features or components may be added.

The sizes of components may be exaggerated or reduced in the accompanying drawings for convenience of description. For example, the size and thickness of each component illustrated in the drawings are arbitrarily indicated for convenience of description, and the present disclosure is not necessarily limited to what is illustrated.

In a case where an embodiment can be implemented differently, the order of specific processes may be performed different from the order in which the processes are described. For example, two processes described in succession may be performed substantially simultaneously, or may be performed in an order opposite to the order described.

In the following embodiments, when components are connected, it includes not only a case where components are directly connected but also a case where components are indirectly connected via certain component interposed between the components. For example, in the present specification, when components and the like are electrically connected, it includes not only a case where components are directly electrically connected, but also a case where components are indirectly electrically connected via certain component interposed between the components.

A method of providing behavioral information for glucose level control by an analyte monitoring system according to an embodiment of the present disclosure includes: acquiring subject glucose level data corresponding to a hypoglycemic range through an analyte monitoring device; acquiring target glucose level data belonging to a normal glucose level range; acquiring a trained neural network model that calculates behavioral information for changing a glucose level value corresponding to the hypoglycemic range to the normal glucose level range; acquiring behavioral information including at least one of a type and amount of food to be consumed by a user from the subject glucose level data and the target glucose level data using the trained neural network model; and outputting the behavioral information through a user terminal device, wherein the trained neural network model may be configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and the trained neural network model may be trained by updating the parameters of the neural network model to output the behavioral prediction value approximated to the behavioral data based on a difference between the behavioral prediction value and the behavioral data.

According to an embodiment of the present disclosure, the outputting of the behavioral information through the user terminal device may further include: acquiring a pre-stored template matching the behavioral information based on the behavioral information; and outputting the behavioral information based on the template.

According to an embodiment of the present disclosure, the method of providing behavioral information may further include: acquiring glucose level data after an action of the user through the analyte monitoring device of the analyte monitoring system; and when the glucose level data after the action reaches the target glucose level data, outputting a notification indicating that the glucose level has returned to the normal range based on the glucose level data after the action and the target glucose level through the user terminal device.

According to an embodiment of the present disclosure, the method of providing behavioral information may further include: when the glucose level data after the action has not reached the target glucose level data, acquiring additional behavioral information including at least one of the type and amount of food to be additionally consumed by the user from the glucose level data after the action and the target glucose level data, using the trained neural network model; and outputting the additional behavioral information through the user terminal device.

According to an embodiment of the present disclosure, the method of providing behavioral information may further include: comparing the glucose level data after the action with the target glucose level data to update the parameters of the trained neural network model.

According to an embodiment of the present disclosure, the updating of the parameters of the trained neural network model may further include updating the parameters of the trained neural network model based on the difference between the glucose level data after the action and the target glucose level data to output the behavioral information so that the glucose level data after the action approximates the target glucose level data.

According to an embodiment of the present disclosure, the outputting of the behavioral information through the user terminal device may further include: outputting at least one piece of selectable behavioral information; acquiring a user input for selecting behavioral information preferred by the user from among the at least one piece of selectable behavioral information through the user terminal device; and inputting preference type information corresponding to the user input as input data of the trained neural network model.

According to an embodiment of the present disclosure, the acquiring of the glucose level data after the action of the user may further include determining whether an action of the user based on the behavioral information has been performed, and the determining of whether the action of the user has been performed may further include: providing an inquiry notification related to whether an action based on the behavioral information has been performed through the user terminal device; acquiring an answer corresponding to the inquiry notification through the user terminal device; and determining whether an action based on the behavioral information has been performed based on the answer.

The acquiring of the glucose level data after the action of the user may further include determining whether an action of the user based on the behavioral information has been performed, and the determining of whether the action of the user has been performed may further include: acquiring change rate information of the glucose level data from the analyte monitoring device; and determining whether an action based on the behavioral information has been performed based on the change rate information and a predetermined change rate value.

According to an embodiment of the present disclosure, a non-transitory computer-readable recording medium on which a computer program for executing the method of providing behavioral information for glucose level control is recorded may be provided.

An analyte monitoring device according to an embodiment of the present disclosure includes: a memory storing at least one instruction; and a processor, wherein the processor may execute the at least one instruction to acquire subject glucose level data corresponding to a hypoglycemic range, acquire target glucose level data belonging to a normal glucose level range, acquire a trained neural network model that calculates behavioral information for changing a glucose level value corresponding to the hypoglycemic range to the normal glucose level range, acquire behavioral information including at least one of a type and amount of food to be consumed by a user from the subject glucose level data and the target glucose level data using the trained neural network model, and transmit the behavioral information, the trained neural network model may be configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and the trained neural network model may be trained by updating the parameters of the neural network model to output the behavioral prediction value approximated to the behavioral data based on a difference between the behavioral prediction value and the behavioral data.

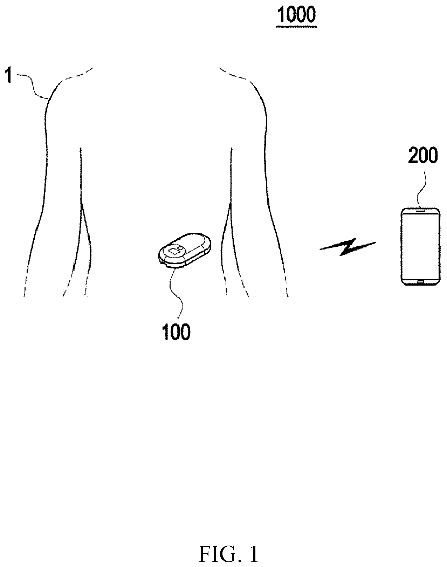

Hereinafter, a method of providing behavioral information for glucose level control, a device for performing the same, and an analyte monitoring system according to an embodiment of the present disclosure will be described in more detail with reference to FIGS. 1 to 12.

FIG. 1 is a schematic diagram illustrating an analyte monitoring system 1000 according to an embodiment of the present disclosure.

Referring to FIG. 1, the analyte monitoring system 1000 may include an analyte monitoring device 100 and a user terminal device 200. For example, the analyte monitoring system 1000 may be a continuous glucose monitoring system (CGMS), and the analyte monitoring device 100 may be a continuous glucose meter (CGM). The user terminal device 200 may be a smart phone, a tablet personal computer (PC), a smart watch, personal digital assistant (PDA), or a dedicated receiver (e.g., receiver).

The analyte monitoring device 100 may be attached to the body of a user 1. At least a portion of the analyte monitoring device 100 may be inserted into a skin of the user 1 and located within the body of the user 1.

The analyte monitoring device 100 may acquire information related to an analyte included in a body fluid of a user 1. The analyte may include glucose and/or a ketone. The information related to the analyte may include any data related to the analyte, including current data related to the analyte, a value representing a concentration of the analyte calculated from the current data, and a glucose level value calculated from the concentration of the analyte.

The user terminal device 200 may receive the information related to the analyte from the analyte monitoring device 100. Furthermore, the user terminal device 200 may provide the information related to the analyte to the user. Specifically, the user terminal device 200 may display the information related to the analyte. The information related to the analyte provided to the user may include data processed by the user terminal device 200. For example, the user terminal device 200 may apply a predefined algorithm to a signal sensed by the analyte monitoring device 100 to generate a glucose concentration value.

Furthermore, the analyte monitoring device 100 or the user terminal device 200 may calculate information on food (or emergency food) to be consumed by the user based on the information related to the analyte. For example, the analyte monitoring device 100 or the user terminal device 200 may calculate information on the food to be consumed by the user in order to return to a normal glucose level range based on the information related to the analyte corresponding to a hypoglycemic range. In this case, the user terminal device 200 may provide the user with the information on the food to be consumed by the user. Specifically, the user terminal device 200 may display the information on the food to be consumed by the user.

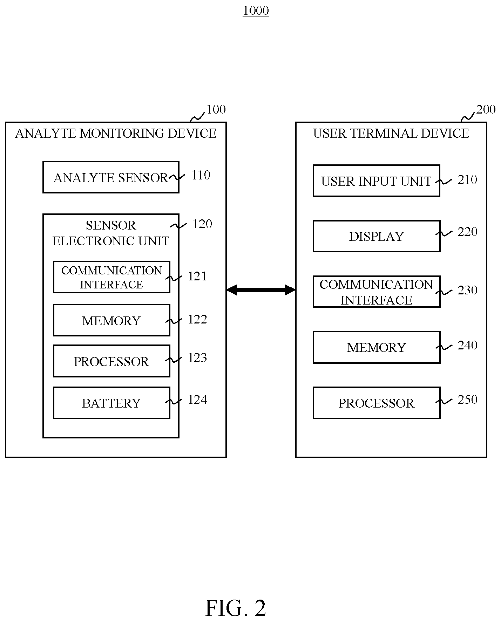

FIG. 2 is a block diagram illustrating a configuration of the analyte monitoring system 1000 according to an embodiment of the present disclosure.

Referring to FIG. 2, the analyte monitoring system 1000 may include the analyte monitoring device 100 and the user terminal device 200.

The analyte monitoring device 100 may include an analyte sensor 110 and a sensor electronic unit 120. The sensor electronic unit 120 may include a communication interface 121, a memory 122, a processor 123, and a battery 124. For example, the analyte monitoring device 100 may be a continuous glucose meter (CGM).

The analyte sensor 110 may be configured to sense an analyte signal. The analyte sensor 110 may include a sensor probe at least partially inserted into the body. A sensing area that reacts with glucose within the body may be formed in the sensor probe to measure a glucose level in the body.

The communication interface 121 includes at least one communication circuit and may communicate with various types of external devices. For example, the communication interface 121 may transmit the information related to the analyte signal or the information on the food to be consumed by the user to the user terminal device 200. The communication interface 121 may include a Bluetooth module, a low-power Bluetooth module, a radio frequency (RF) module, and/or a near field communication (NFC) module.

The memory 122 may store an operating system (OS) for controlling the overall operation of the components of the analyte monitoring device 100 and commands or data related to the components of the analyte monitoring device 100. In particular, the memory 122 may store instructions for calculating behavioral information, which will be described below. The memory 122 may be implemented as a non-volatile memory (e.g., a hard disk, a solid state drive (SSD), and a flash memory), a volatile memory, or the like.

The processor 123 may be electrically connected to the memory 122 to control the overall function and operation of the analyte monitoring device 100. The processor 123 may control the overall operation of the analyte monitoring device 100 by executing the instructions stored in the memory 122.

The battery 124 may provide power to the components of the analyte monitoring device 100.

The user terminal device 200 may include a user input unit 210, a display 220, a communication interface 230, a memory 240, and a processor 250. For example, the user terminal device 200 may be a smartphone.

The user input unit 210 is configured to receive user input or user commands. The user terminal device 200 may receive any appropriate user input or user command through the user input unit 210, including a user input for selecting behavioral information preferred by the user from among at least one piece of behavioral information that can be selected, which will be described later and/or a user input for answers corresponding to inquiries related to whether the user's action has been performed.

The user input unit 210 may be configured to include a key pad, a dome switch, a touch pad (static pressure/electrostatic), a jog wheel, a jog switch, and a sensor (e.g., a voice sensor, a proximity sensor, an illuminance sensor, an acceleration sensor, a gyro sensor, etc.). The user input unit 210 may be implemented in the form of a button on the outside of the user terminal device 200, and some buttons may be implemented as a touch panel. When a user input (e.g., text input, voice input, change in user device operation, etc.) for rule definition and rule execution (e.g., command) is received, the user input unit 210 may generate the input data accordingly.

The display 220 may output the information related to the analyte signal. For example, the display 220 may output the glucose level value of the user. In addition, the display 220 may output the behavioral information. For example, the display 220 may output the behavioral information on the type or amount of food to be consumed by the user. In addition, the display 220 may output UI elements for receiving a user input. For example, the display 220 may output UI elements for receiving the user input for selecting the behavioral information preferred by a user from among at least one piece of behavioral information that can be selected, which will be described later, and/or the user input for answers corresponding to inquiries related to whether the user's action has been performed.

The communication interface 230 may include at least one communication circuit. The communication interface 230 may receive the information related to the analyte signal from the communication interface 121 of the analyte monitoring device 100. The communication interface 230 may include a Bluetooth module, a low-power Bluetooth module, a radio frequency (RF) module, and/or a near field communication (NFC) module.

The memory 240 may store the operating system (OS) for controlling the overall operation of the components of the user terminal device 200 and commands or data related to the components of the user terminal device 200. In particular, the memory 240 may store instructions for calculating behavioral information, which will be described later, or for displaying the calculated behavioral information. The memory 250 may be implemented as a non-volatile memory (e.g., a hard disk, a solid state drive (SSD), and a flash memory), a volatile memory, or the like.

The processor 250 may be electrically connected to the memory 240 to control the overall function and operation of the user terminal device 200. The processor 250 may execute instructions stored in the memory 240 to control the user terminal device 200. For example, the processor 250 may control the display 220 to display the information related to the analyte signal and/or the behavioral information.

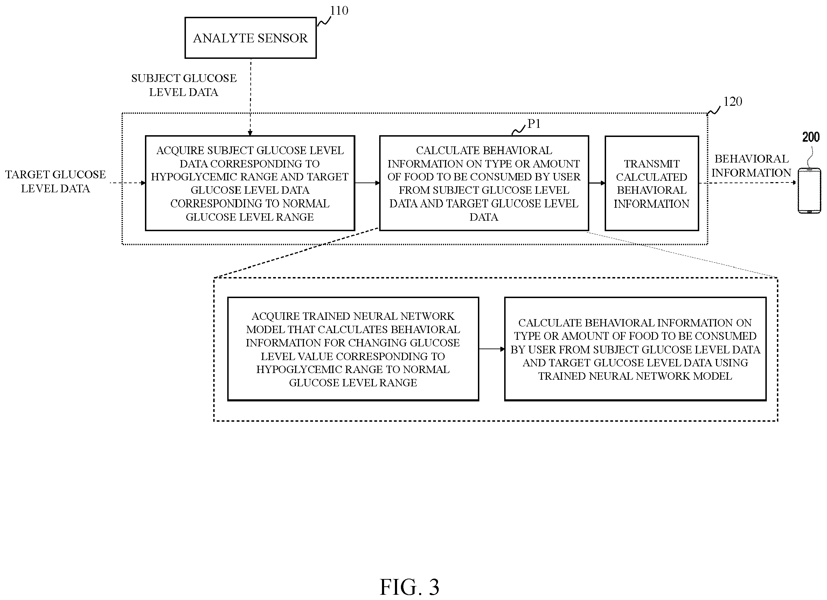

FIG. 3 is a diagram for describing the operation of the analyte monitoring system 1000 and/or the analyte monitoring device 100 according to an embodiment of the present disclosure.

The analyte monitoring system 1000 according to an embodiment of the present disclosure may perform an operation of monitoring glucose level data and calculating behavioral information related to the food to be consumed to adjust the glucose level corresponding to the hypoglycemic range to the glucose level of the normal glucose level range.

Specifically, the analyte monitoring device 100 of the analyte monitoring system 1000 may acquire data (hereinafter referred to as subject glucose level data) related to an analyte (e.g., glucose) included in the body fluid of the user 1 from the analyte sensor 110. Data related to the analyte may include current data detected by an electrode of the analyte sensor 110, concentration data calculated based on the current data, and/or glucose level value data calculated based on the concentration data. According to an embodiment, the subject glucose level data may be data related to the analyte corresponding to the hypoglycemic range.

Furthermore, the analyte monitoring device 100 of the analyte monitoring system 1000 may acquire target glucose level data. The target glucose level data may include current data corresponding to the glucose level value, analyte concentration data, and/or glucose level value data that belongs to a target glucose level range. According to an embodiment, the target glucose level data may be data corresponding to a glucose level value belonging to a normal glucose level range.

The analyte monitoring device 100 according to an embodiment of the present disclosure may perform an operation P1 of calculating behavioral information on the type, amount, or number of foods to be consumed by the user from the subject glucose level data and the target glucose level data. The behavioral information may encompass any information that directs a user's action related to food to be consumed, including the type, amount, and/or number of foods to be consumed.

According to an embodiment, the behavioral information calculation operation P1 may be performed using a trained neural network model. Specifically, the analyte monitoring device 100 may acquire a trained neural network model to change the glucose level value corresponding to the hypoglycemic range to the glucose level value belonging to the normal glucose level range. Acquiring the trained neural network model means acquiring arbitrary execution data to properly execute the trained neural network model, including layer information, operation library information, and/or parameter information of the trained neural network model. Furthermore, the analyte monitoring device 100 may calculate the behavioral information on the type or amount of food to be consumed by the user from the subject glucose level data and the target glucose level data using the trained neural network model.

The trained neural network model according to an embodiment may be configured to output a behavioral prediction value that approximates behavioral data related to the type or amount of food consumed by the user based on training data that is composed of first glucose level data belonging to the hypoglycemic range, behavioral data related to the type or amount of food consumed by the user, and/or second glucose level data belonging to the normal glucose level range after the action corresponding to the behavior data. Therefore, the trained neural network model may use the subject glucose level data belonging to the hypoglycemic range and the target glucose level data belonging to the normal glucose level range as input data to calculate the behavioral information on the type or amount of food to be consumed by the user.

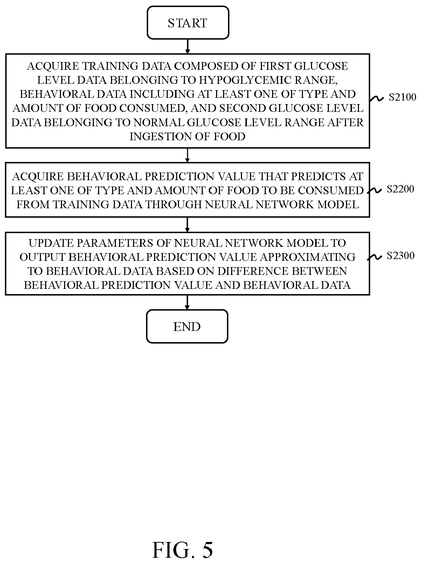

The training method of the neural network model and the details of calculating behavioral information using the trained neural network model will be described in more detail with reference to FIGS. 5 to 8.

The analyte monitoring device 100 according to an embodiment of the present disclosure may transmit the calculated behavioral information to the user terminal device 200 through the communication interface 121. The user terminal device 200 may receive the behavioral information through the communication interface 230. Furthermore, the user terminal device 200 may display the received behavioral information to the user through the display 220.

Meanwhile, in FIG. 3, it is described that the behavioral information calculation operation P1 is performed by the analyte monitoring device 100. However, this is only for convenience of description, and the behavioral information calculation operation P1 may be performed by the user terminal device 200. For example, the user terminal device 200 may acquire the target glucose level data belonging to the normal glucose level range through the communication interface 230 and acquire the subject glucose level data corresponding to the hypoglycemic range from the analyte sensor 110. Furthermore, the user terminal device 200 may acquire the trained neural network model and acquire the behavioral information on the type or amount of food to be consumed by the user from the subject glucose level data and the target glucose level data through the trained neural network model. Furthermore, the user terminal device 200 may display the behavioral information to the user through the display 220.

Hereinafter, for convenience of description, it will be described that the behavioral information calculation operation P1 is performed by the analyte monitoring device 100. However, this is only for convenience of description and should not be construed as being limited thereto.

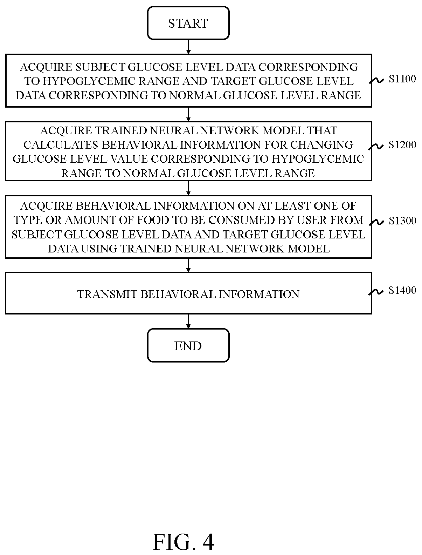

Hereinafter, with reference to FIGS. 4 to 12, the analyte monitoring device 100 and the method of providing behavioral information for glucose level control performed by the analyte monitoring device 100 according to an embodiment of the present disclosure will be described in more detail. Meanwhile, when describing the method of providing behavioral information for glucose level control, some embodiments that overlap with the content previously described with reference to FIGS. 1 to 3 may be omitted. However, this is only for convenience of description and should not be construed as being limited thereto.

FIG. 4 is a flowchart illustrating a method of providing behavioral information for glucose level control according to an embodiment of the present disclosure.

A method of providing behavioral information for glucose level control according to an embodiment of the present disclosure includes acquiring target glucose level data corresponding to a hypoglycemic range and target glucose level data corresponding to a normal glucose level range (S 11 00), acquiring a trained neural network model that calculates the behavioral information for changing a glucose level value corresponding to the hypoglycemic range to the normal glucose level range (S1200), acquiring behavioral information including at least one of a type and amount of food to be consumed by a user from the subject glucose level data and the target glucose level data using the trained neural network model (S1300), and/or transmitting the behavioral information (S1400).

In the operation (S 1100) of acquiring the subject glucose level data corresponding to the hypoglycemic range and the target glucose level data corresponding to the normal glucose level range, the analyte monitoring device 100 may acquire data (i.e., subject glucose level data) related to the analyte (e.g., glucose) contained in a body fluid of a user from the analyte sensor 110 through the communication interface 121. The data related to the analyte may include current data detected by an electrode of the analyte sensor 110, concentration data calculated based on the current data, and/or glucose level value data calculated based on the concentration data. According to an embodiment, the subject glucose level data may be data related to an analyte corresponding to the hypoglycemic range.

Furthermore, in the acquiring of the subject glucose level data corresponding to the hypoglycemic range and the target glucose level data corresponding to the normal glucose level range (S1100), the analyte monitoring device 100 may acquire the target glucose level data through the communication interface 121. The target glucose level data may include current data corresponding to the glucose level value, the analyte concentration data, and/or the glucose level value data that belongs to the target glucose level range. According to an embodiment, the target glucose level data may be data corresponding to a glucose level value belonging to the normal glucose level range.

In the acquiring of the trained neural network model that calculates the behavioral information for changing the glucose level value corresponding to the hypoglycemic range to the normal glucose level range (S1200), the analyte monitoring device 100 may acquire a trained neural network model for calculating behavioral information for changing the glucose level value corresponding to the hypoglycemic range to the normal glucose level range through the communication interface 121. Specifically, the analyte monitoring device 100 may be configured to acquire the trained neural network model through the communication interface 121.

The behavioral information may encompass any information that directs a user's action related to food to be consumed, including the type, amount, and/or number of foods to be consumed.

Acquiring the trained neural network model means acquiring arbitrary execution data to properly execute the trained neural network model, including layer information, operation library information, and/or parameter information of the trained neural network model.

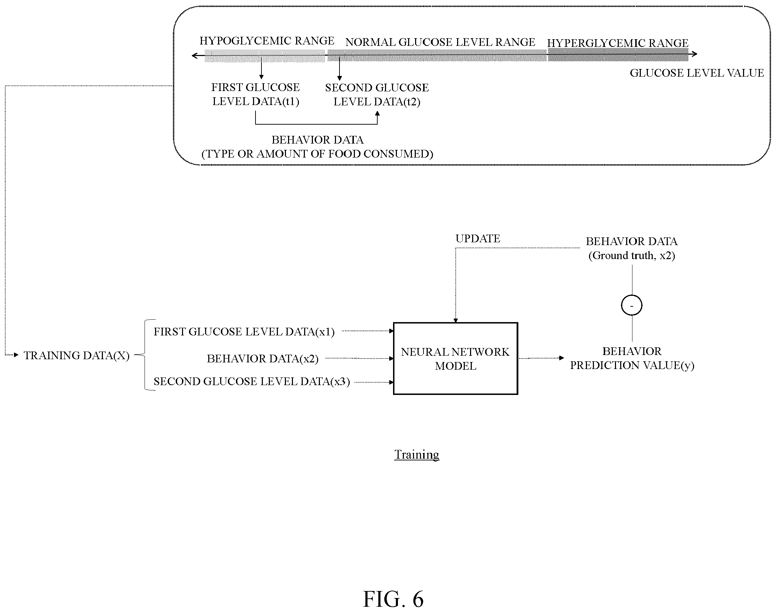

Hereinafter, the method of training a neural network model according to an embodiment of the present disclosure will be described in more detail with reference to FIGS. 5 and 6. The training of the neural network model may be performed in the analyte monitoring device 100, the user terminal device 200, and/or an external electronic device (or an external server). Hereinafter, the training process of the neural network model will be described as being performed in an external electronic device. However, this is only an example, and should not be construed as being limited thereto.

FIG. 5 is a flowchart illustrating a method of training a neural network model configured to calculate behavioral information for glucose level control according to an embodiment of the present disclosure. FIG. 6 is a diagram illustrating an aspect of training a neural network model according to an embodiment of the present disclosure.

The method of training a neural network model according to an embodiment of the present disclosure may include acquiring training data composed of first glucose level data belonging to the hypoglycemic range, behavioral data including at least one of a type and amount of food consumed, and second glucose level data belonging to the normal glucose level range after consumption of the food (S2100), acquiring a behavioral prediction value that predicts at least one of the type and amount of food consumed from the training data through a neural network model (S2200), and/or updating parameters of the neural network model to output a behavioral prediction value approximating to the behavioral data based on a difference between the behavioral prediction value and the behavioral data (S2300).

In the acquiring of the training data composed of the first glucose level data belonging to the hypoglycemic range, the behavioral data including at least one of the type and amount of food consumed, and the second glucose level data belonging to the normal glucose level range after consumption of the food (S2100), the electronic device may acquire training data to train the neural network model for calculating behavioral information. The training data may be composed of first glucose level data belonging to the hypoglycemic range at a first time point t1 (e.g., the first glucose level data may be in the form of a hypoglycemia value belonging to the hypoglycemic range, the analyte concentration data corresponding to the hypoglycemia value, and /or the current data corresponding to the hypoglycemia value), the behavioral data on the type (e.g., candy, chocolate, juice, soda, yogurt, honey, and/or sugar) and/or amount of food consumed, and/or the second glucose level data (e.g., the second glucose level data may be in the form of a normal glucose level value belonging to the normal glucose level range, the analyte concentration data corresponding to the normal glucose level value, and/or the current data corresponding to the normal glucose level value) at a second time point t2 after consumption of the food.

In the acquiring of the behavioral prediction value that predicts at least one of the type and amount of food consumed from the training data through the neural network model (S2200), the electronic device may be configured to input first glucose level data x1, behavioral data x2, and second glucose level data x3 of training data X to the neural network model, and acquire a behavioral prediction value y that predicts the type or amount of food consumed from the behavioral data x2 included in the training data output through the neural network model.

In the updating of the parameters of the neural network model to output the behavioral prediction value approximating to the behavioral data based on the difference between the behavioral prediction value and the behavioral data (S2300), the electronic device may be configured to update the parameters of the neural network model to output a behavioral prediction value approximating the behavioral data based on the difference between the behavioral prediction value y and the behavioral data x2 acquired through operation S2200. Specifically, the electronic device may use the behavioral data x2 of the training data as ground truth to update the parameters of the neural network model so that the neural network model calculates a behavioral prediction value approximating the ground truth from the first glucose level data corresponding to the hypoglycemic range and the second glucose level data corresponding to the normal glucose level range.

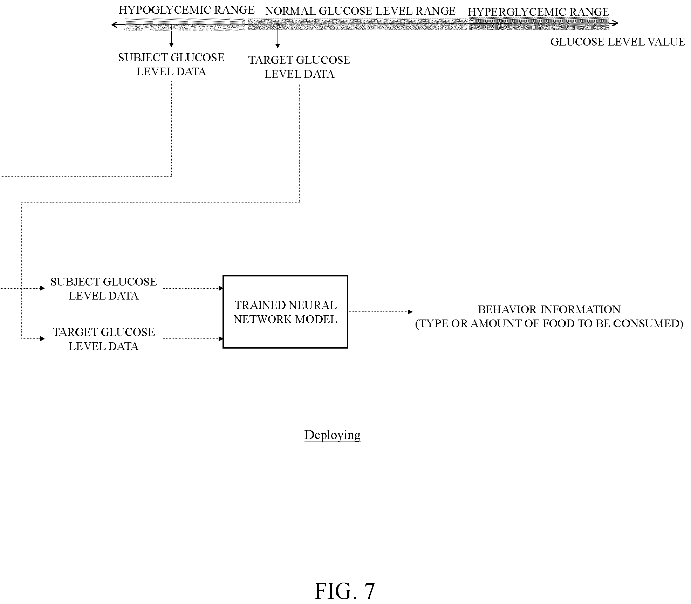

FIG. 7 is a diagram illustrating an aspect of calculating behavioral information using a trained neural network model according to an embodiment of the present disclosure.

The neural network model that has been trained according to the method of training a neural network model according to an embodiment of the present disclosure may be configured to calculate the behavioral information on the type and/or amount of food to be consumed based on the subject glucose level data corresponding to the hypoglycemic range and the target glucose level data corresponding to the normal glucose level range. Specifically, since the neural network model is trained to output a behavioral prediction value approximating the behavioral data on the type and/or amount of food consumed based on the first glucose level data corresponding to the hypoglycemic range and the second glucose level data corresponding to the normal glucose level range, the trained neural network model may output the behavioral information on the type and/or amount of food to be consumed based on the subject glucose level data of the hypoglycemic range corresponding to the first glucose level data and the target glucose level data of the normal glucose level range corresponding to the second glucose level data.

Referring back to FIG. 4, the analyte monitoring device 100 according to an embodiment of the present disclosure may acquire the behavioral information on at least one of the type and amount of food to be consumed by the user from the subject glucose level data and the target glucose level data using the trained neural network model (S1300).

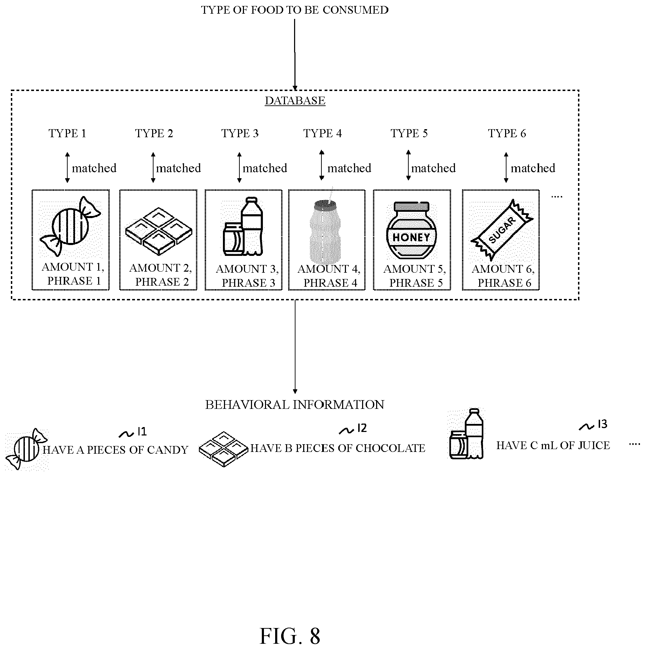

FIG. 8 is a diagram illustrating an aspect of outputting a template corresponding to behavioral information according to an embodiment of the present disclosure.

The analyte monitoring system 1000 according to an embodiment of the present disclosure may be configured to acquire a template corresponding to the type of food in behavioral information and output behavioral information through the user terminal device 200 based on the template.

Specifically, templates (e.g., phrases, predetermined amounts, icons, etc.) matching each type of food may be pre-stored in a database. In this case, the analyte monitoring device 100 or the user terminal device 200 of the analyte monitoring system 1000 may be configured to acquire the template corresponding to the type of food in the behavioral information from the database and output behavioral information based on the acquired template. For example, when the behavioral information acquired through the trained neural network model includes a first type (e.g., candy) as the type of food to be consumed, the analyte monitoring device 100 or the user terminal device 200 may acquire a first template (e.g., phrase 1, amount 1 (A pieces), candy icon) matching the first type from the database and output behavioral information (e.g., I1 in FIG. 8) based on the first template. For example, when the behavioral information acquired through the trained neural network model includes a second type (e.g., chocolate) as the type of food to be consumed, the analyte monitoring device 100 or the user terminal device 200 may acquire a second template (e.g., phrase 2, amount 2 (B pieces), chocolate icon) matching the second type from the database and output behavioral information (e.g., I2 in FIG. 8) based on the second template. For example, when the behavioral information acquired through the trained neural network model includes a third type (e.g., juice or soda) as the type of food to be consumed, the analyte monitoring device 100 or the user terminal device 200 may acquire a third template (e.g., phrase 3, amount 3 (C mls), juice icon or soda icon) matching the third type from the database and output behavioral information (e.g., 13 in FIG. 8) based on the third template. For example, when the behavioral information acquired through the trained neural network model includes a fourth type (e.g., yogurt) as the type of food to be consumed, the analyte monitoring device 100 or the user terminal device 200 may acquire a fourth template (e.g., phrase 4, amount 4, yogurt icon) matching the fourth type from the database and output behavioral information based on the fourth template. For example, when the behavioral information acquired through the trained neural network model includes a fifth type (e.g., honey) as the type of food to be consumed, the analyte monitoring device 100 or the user terminal device 200 may acquire a fifth template (e.g., phrase 5, amount 5, honey icon) matching the fifth type from the database and output behavioral information based on the fifth template. For example, when the behavioral information acquired through the trained neural network model includes a sixth type (e.g., sugar) as the type of food to be consumed, the analyte monitoring device 100 or the user terminal device 200 may acquire a sixth template (e.g., phrase 6, amount 6, sugar icon) matching the sixth type from the database and output behavioral information based on the sixth template.

Meanwhile, in FIG. 8, the description focuses on a specific type or specific template. However, this is only an example, and the analyte monitoring system 1000 according to an embodiment of the present disclosure may be configured to output behavioral information using any type of appropriate food and any appropriate template matched by type.

Referring back to FIG. 4, the analyte monitoring device 100 according to an embodiment of the present disclosure may transmit the behavioral information acquired through operation S1300 (S1400). More specifically, the analyte monitoring device 100 may transmit the behavioral information to the user terminal device 200 through the communication interface 121.

The user terminal device 200 may be configured to receive the behavioral information through the communication interface 230 and output the received behavioral information through the display 220.

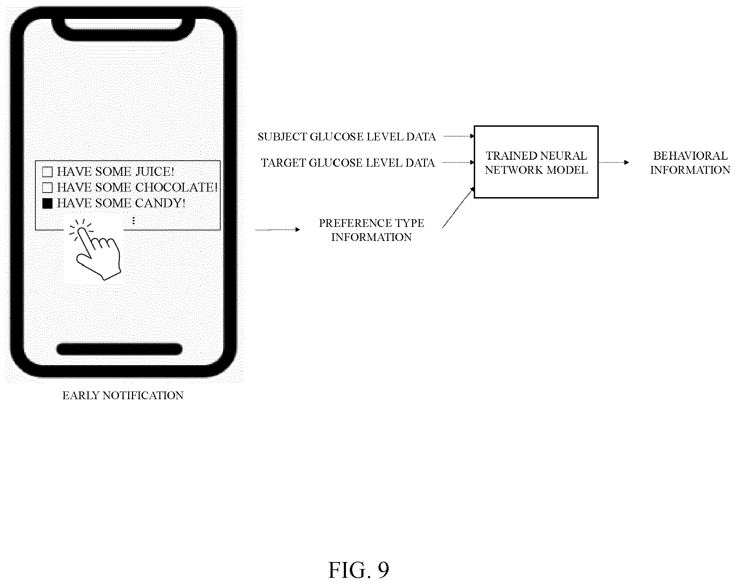

FIG. 9 is a diagram illustrating an aspect of calculating behavioral information based on preference type information according to an embodiment of the present disclosure.

The analyte monitoring system 1000 according to an embodiment of the present disclosure may be configured to provide at least one piece of selectable behavioral information. Specifically, when behavioral information is provided to the user for the first time, the analyte monitoring system 1000 may provide the user with at least one piece of selectable behavioral information (e.g., refer to the initial notification in FIG. 9) through the user terminal device 200. For example, the analyte monitoring system 1000 may provide a user with first behavioral information corresponding to a first type (e.g., candy) of food and second behavioral information corresponding to a second type (e.g., juice, chocolate, or the like) of food through the user terminal device 200.

In this case, the user may select a preferred type of food from among at least one piece of selectable behavioral information through the user input unit 210 of the user terminal device 200. Furthermore, the user terminal device 200 may transmit the preference type information to the analyte monitoring device 100 through the communication interface 230 based on the type of food selected. The analyte monitoring device 100 may be configured to receive the preference type information through the communication interface 121 and input the received preference type information as input data of the trained neural network model. That is, a neural network model whose training has been completed after receiving the preference type information may be configured to calculate behavioral information based on the subject glucose level data, the target glucose level data, and the preference type information. According to the present embodiment, it is possible to provide an advantageous effect of calculating behavioral information by reflecting the food preference of the user.

Meanwhile, in FIG. 9, it is described that the preference type information is obtained by selecting behavioral information corresponding to a specific type of food from among at least one piece of selectable behavioral information. However, this is only an example, and the analyte monitoring system 1000 may be configured to receive information on the type of preferred food directly from the user and calculate behavioral information based on the information on the type of preferred food through the trained neural network model.

According to an embodiment of the present disclosure, the analyte monitoring system 1000 may be configured to perform an operation of providing a notification indicating that the glucose level data after the action has reached the target glucose level data or calculating additional behavioral information, based on the target glucose level data and the glucose level data after the action.

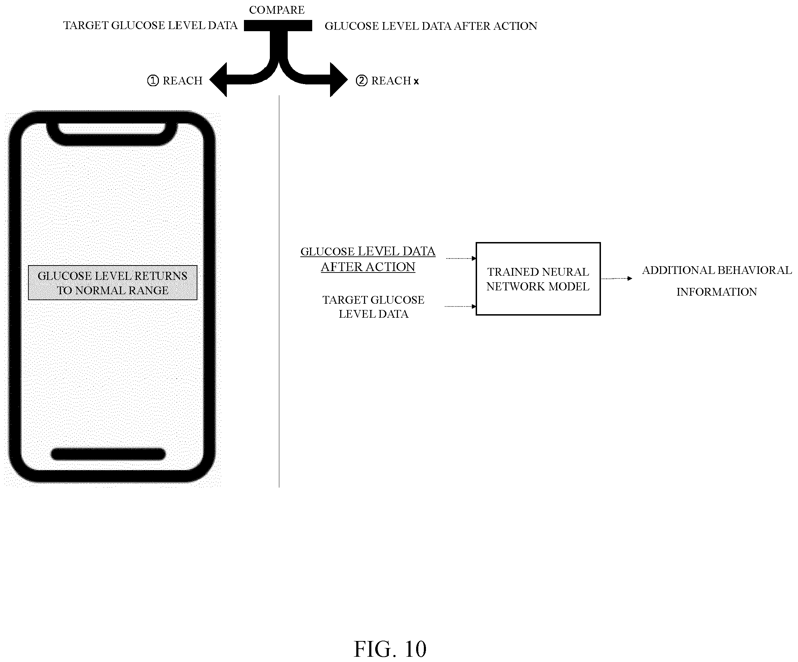

FIG. 10 is a diagram illustrating an aspect of providing a notification indicating that the glucose level has returned to a normal range or calculating additional behavioral information, depending on whether glucose level data after the action has reached target glucose level data according to an embodiment of the present disclosure.

As described above, the analyte monitoring device 100 according to an embodiment of the present disclosure may acquire behavioral information on the type or amount of food to be consumed from the subject glucose level data and the target glucose level data using the trained neural network model. Furthermore, the analyte monitoring device 100 may acquire glucose level data (hereinafter referred to as the glucose level data after the action) after an action based on the behavioral information has been performed. In this case, the analyte monitoring device 100 may compare the target glucose level data and the glucose level data after the action.

For example, by comparing the target glucose level data with the glucose level data after the action, when the glucose level data after the action reaches the target glucose level data, the analyte monitoring device 100 may be configured to output a notification indicating that the glucose level has returned to the normal range through the user terminal device 200.

For example, by comparing the target glucose level data with the glucose level data after the action, when the glucose level data after the action has not reached the target glucose level data, the analyte monitoring device 100 may be configured to acquire additional behavioral information using the trained neural network model. Specifically, when the glucose level data after the action has not reached the target glucose level data, the analyte monitoring device 100 may be configured to input the glucose level data after the action and the target glucose level data to the trained neural network model and acquire additional behavioral information on the type or amount of food to be additionally consumed by the user calculated from the glucose level data after the action and the target glucose level data through the trained neural network model.

According to the present embodiment, it is possible to improve user convenience and usability by notifying the user that the glucose level has reached the normal range. Furthermore, according to the present embodiment, it is possible to prevent shock due to hypoglycemia by providing additional behavioral information to the user.

According to an embodiment of the present disclosure, the trained neural network model may be additionally updated during the execution process (or deployment process).

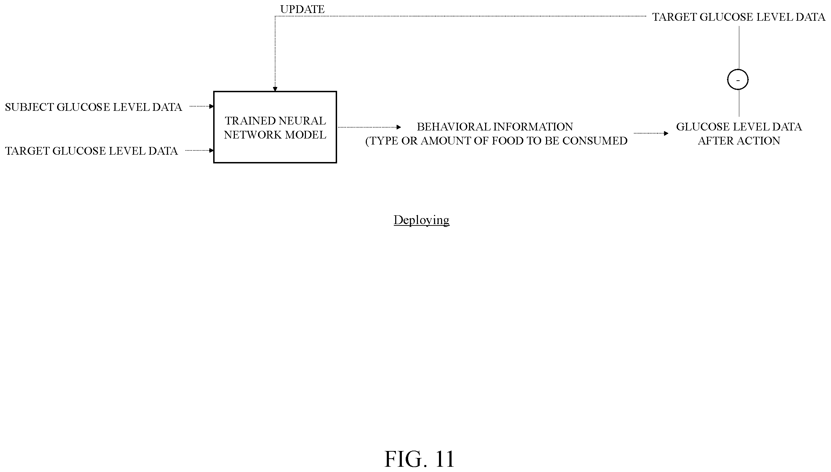

FIG. 11 is a diagram illustrating an aspect of training a neural network model whose training has been completed in a process of executing the neural network model according to an embodiment of the present disclosure.

As described above, the analyte monitoring device 100 may acquire the glucose level data after the action. In this case, the analyte monitoring device 100 may compare the glucose level data after the action and the target glucose level data to perform the operation of updating the parameters of the trained neural network model. Specifically, the analyte monitoring device 100 may be configured to update the parameters of the trained neural network model based on the difference between the glucose level data after the action and the target glucose level data to output the behavioral information for the glucose level data after the action to approximate the target glucose level data. According to the present embodiment, it is possible to provide a neural network model that calculates more precise behavioral information to reach the target glucose level data.

Meanwhile, although not illustrated in FIG. 11, the analyte monitoring system 1000 according to an embodiment of the present disclosure may be configured to additionally update the parameters of the trained neural network model based on the difference between the target glucose level data and the glucose level data after the additional action based on the additional behavioral information in FIG. 10.

According to an embodiment of the present disclosure, the analyte monitoring system 1000 may be configured to determine whether an action of the user based on the behavioral information has been performed.

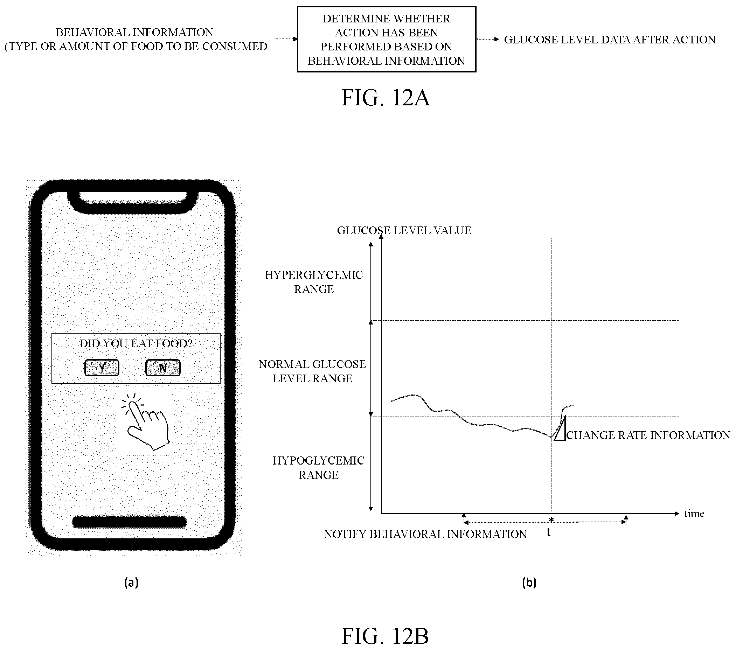

FIGS. 12A and 12B are diagrams for describing aspects of determining whether an action based on behavioral information has been performed according to an embodiment of the present disclosure.

According to an embodiment, the user terminal device 200 of the analyte monitoring system 1000 may provide an inquiry notification indicating whether an action based on the behavioral information has been performed through the display 220. In this case, the user may input an answer corresponding to the inquiry notification through the user input unit 210 of the user terminal device 200. In this case, the analyte monitoring device 100 or the user terminal device 200 of the analyte monitoring system 1000 may determine whether an action based on the behavioral information has been performed based on the answer. For example, when the answer is that food has been consumed, the analyte monitoring device 100 or the user terminal device 200 of the analyte monitoring system 1000 may determine that an action based on the behavioral information has been performed based on the answer. For example, when the answer is that food has not been consumed, the analyte monitoring device 100 or the user terminal device 200 of the analyte monitoring system 1000 may determine that an action based on the behavioral information has not been performed based on the answer.

According to an embodiment, the analyte monitoring system 1000 may acquire change rate information of the glucose level data. In this case, the analyte monitoring device 100 and the user terminal device 200 of the analyte monitoring system 1000 may determine whether an action based on the behavioral information has been performed based on the change rate information and a predetermined change rate value. For example, when the change rate information of the glucose level data (e.g., glucose level value) is greater than or equal to the predetermined change rate value within a predetermined time period t after the notification of the behavioral information is provided, the analyte monitoring device 100 and the user terminal device 200 of the analyte monitoring system 1000 may determine that an action based on the behavioral information has been performed. For example, when the change rate information is smaller than the predetermined change rate value within the predetermined time period t after the notification of the behavioral information is provided, the analyte monitoring device 100 and the user terminal device 200 of the analyte monitoring system 1000 may determine that an action based on the behavioral information has not been performed. Meanwhile, in FIG. 12B, it is described that the change rate of the glucose level value over time is used to determine whether an action based on the behavioral information has been performed. However, this is only an example, and it may be configured to determine whether an action based on the behavioral information has been performed using the change rate of the current value over time or the change rate of the concentration value of the analyte over time.

Meanwhile, when it is determined that an action based on the behavioral information has been performed, the analyte monitoring system 1000 may acquire the glucose level data after the action through the analyte monitoring device 100. On the other hand, when the analyte monitoring system 1000 determines that an action based on the behavioral information has not been performed by the predetermined time t after the notification of the behavioral information is provided, the analyte monitoring system 1000 may be configured to re-output behavioral information through the display 220 of the user terminal device 200.

According to the present embodiment, by determining whether the action of consuming food has been performed, it is possible to provide the advantageous effect of lowering the possibility of shock due to hypoglycemia that can occur due to not eating food.

The analyte monitoring system 1000 according to an embodiment of the present disclosure may update health information related to the glucose level of the user based on the notification frequency of behavioral information. Specifically, when the notification frequency of behavioral information is relatively high, it may be understood that the frequency of occurrence of hypoglycemia is relatively high. Accordingly, the analyte monitoring system 1000 may be configured to update a user's health status to a hypoglycemia risk group when the notification frequency is greater than a predetermined threshold frequency based on the notification frequency of behavioral information.

Furthermore, the analyte monitoring system 1000 according to the embodiment of the present disclosure may acquire the user's pattern information on meal time, exercise time, bedtime, and/or wake-up time, etc., and provide a notification in consideration of the user's pattern information. For example, when glucose level data corresponding to the hypoglycemic range is acquired adjacent to meal time (e.g., when the difference between the current time and the meal time is within a predetermined time), the analyte monitoring system 1000 may be configured to provide a reminder to eat, instead of a reminder to consume food.

According to the method for providing behavioral information for glucose level control, the device for performing the same, and the analyte monitoring system according to an embodiment of the present disclosure, by using the information on the type of food the user prefers as input data of the neural network model, it is possible to provide an advantageous effect of calculating behavioral information by reflecting the user's food preference.

According to the method for providing behavioral information for glucose level control, the device for performing the same, and the analyte monitoring system according to an embodiment of the present disclosure, by updating the trained neural network model during the execution process based on the glucose level data after an action and the target glucose level data, it is possible to provide a neural network model that calculates more precise behavioral information to reach the target normal glucose level.

According to the method for providing behavioral information for glucose level control, the device for performing the same, and the analyte monitoring system according to an embodiment of the present disclosure, by determining whether the user has consumed food and calculating additional behavioral information, it is possible to provide an advantageous effect of lowering the possibility of shock due to hypoglycemia that can occur due to the user not eating food.

The effects of the present disclosure are not limited to the above-described effects, and effects that are not mentioned will be clearly understood by those skilled in the art to which the present disclosure pertains from the present specification and the accompanying drawings.

The features, structures, effects, etc., described in the above embodiments are included in at least one embodiment of the present disclosure, and are not necessarily limited only to one embodiment. Furthermore, the features, structures, effects, etc., illustrated in each embodiment can be combined or modified and implemented in other embodiments by those of ordinary skill in the art to which the embodiments pertain. Accordingly, the contents related to such combinations and modifications should be construed as being included in the scope of the present disclosure.

Although one exemplary embodiment of the present disclosure has been mainly described hereinabove, this is only an example and does not limit the present disclosure. Those skilled in the art to which the present disclosure pertains may understand that several modifications and applications that are not described in the present specification may be made without departing from the spirit of the present disclosure. That is, each component specifically shown in the embodiment may be modified and implemented. In addition, differences associated with these modifications and applications are to be construed as being included in the scope of the present specification as defined by the following claims.

## Claims

1. A method of providing behavioral information for glucose level control by an analyte monitoring system including an analyte monitoring device for detecting an analyte signal related to a glucose concentration and a user terminal device, the method comprising:
acquiring subject glucose level data corresponding to a hypoglycemic range through the analyte monitoring device;
acquiring target glucose level data belonging to a normal glucose level range;
acquiring a trained neural network model that calculates behavioral information for changing a glucose level value corresponding to the hypoglycemic range to the normal glucose level range;
acquiring behavioral information including at least one of a type and amount of food to be consumed by a user from the subject glucose level data and the target glucose level data using the trained neural network model; and
outputting the behavioral information through the user terminal device,
wherein the trained neural network model is configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and
the trained neural network model is trained by updating parameters of the neural network model to output the behavioral prediction value approximated to the behavioral data based on a difference between the behavioral prediction value and the behavioral data.

2. The method of claim 1, wherein the outputting of the behavioral information through the user terminal device includes:
acquiring a pre-stored template matching the behavioral information based on the behavioral information; and
outputting the behavioral information based on the template.

3. The method of claim 1, further comprising:
acquiring glucose level data after an action of the user through the analyte monitoring device of the analyte monitoring system; and
when the glucose level data after the action reaches the target glucose level data, outputting a notification indicating that the glucose level has returned to the normal range through the user terminal device based on the glucose level data after the action and the target glucose level.

4. The method of claim 3, further comprising:
when the glucose level data after the action has not reached the target glucose level data, acquiring additional behavioral information including at least one of the type and amount of food to be additionally consumed by the user from the glucose level data after the action and the target glucose level data, using the trained neural network model; and
outputting the additional behavioral information through the user terminal device.

5. The method of claim 3, further comprising: comparing the glucose level data after the action with the target glucose level data to update the parameters of the trained neural network model.

6. The method of claim 5, wherein the updating of the parameters of the trained neural network model includes updating the parameters of the trained neural network model based on the difference between the glucose level data after the action and the target glucose level data to output the behavioral information so that the glucose level data after the action approximates the target glucose level data.

7. The method of claim 1, wherein the outputting of the behavioral information through the user terminal device includes:
outputting at least one piece of selectable behavioral information;
acquiring a user input for selecting behavioral information preferred by the user from among the at least one piece of selectable behavioral information through the user terminal device; and
inputting preference type information corresponding to the user input as input data of the trained neural network model.

8. The method of claim 3, wherein the acquiring of the glucose level data after the action of the user includes determining whether the action of the user based on the behavioral information has been performed, and
the determining of whether the action of the user has been performed includes:
providing an inquiry notification related to whether an action based on the behavioral information has been performed through the user terminal device;
acquiring an answer corresponding to the inquiry notification through the user terminal device; and
determining whether an action based on the behavioral information has been performed based on the answer.

9. The method of claim 3, wherein the acquiring of the glucose level data after the action of the user further includes determining whether an action of the user based on the behavioral information has been performed, and
the determining of whether the action of the user has been performed includes:
acquiring change rate information of the glucose level data from the analyte monitoring device; and
determining whether an action based on the behavioral information has been performed based on the change rate information and a predetermined change rate value.

10. A non-transitory computer-readable recording medium on which a computer program executed by a computer is recorded, the computer program comprising:
acquiring subject glucose level data corresponding to a hypoglycemic range through the analyte monitoring device;
acquiring target glucose level data belonging to a normal glucose level range;
acquiring a trained neural network model that calculates behavioral information for changing a glucose level value corresponding to the hypoglycemic range to the normal glucose level range;
acquiring behavioral information including at least one of a type and amount of food to be consumed by a user from the subject glucose level data and the target glucose level data using the trained neural network model; and
outputting the behavioral information through the user terminal device,
wherein the trained neural network model is configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and
the trained neural network model is trained by updating parameters of the neural network model to output the behavioral prediction value approximated to the behavioral data based on a difference between the behavioral prediction value and the behavioral data.

11. An analyte monitoring device, comprising:
a memory configured to store at least one instruction; and
a processor,
wherein the processor executes the at least one instruction to acquire subject glucose level data corresponding to a hypoglycemic range, acquire target glucose level data belonging to a normal glucose level range, acquire a trained neural network model that calculates behavioral information for changing a glucose level value corresponding to the hypoglycemic range to the normal glucose level range, acquire behavioral information including at least one of a type and amount of food to be consumed by a user from the subject glucose level data and the target glucose level data using the trained neural network model, and transmit the behavioral information,
the trained neural network model is configured to calculate a behavioral prediction value, which predicts at least one of the type and amount of food consumed, from training data composed of first glucose level data corresponding to the hypoglycemic range, behavioral data including at least one of the type and amount of food consumed, and second glucose level data belonging to the normal glucose level range after consumption, and
the trained neural network model is trained by updating parameters of the neural network model to output the behavioral prediction value approximated to the behavioral data based on a difference between the behavioral prediction value and the behavioral data.
